# EUROPEAN PATENT SPECIFICATION

(11) **EP 3 558 425 B1**
(45) Date of publication and mention of the grant of the patent: **01.02.2023**
(21) Application number: 17828736.3
(22) Date of filing: 21.12.2017
(51) Int. Cl.: A61M 5/20, A61M 5/30, A61B 17/20

(54) **NEEDLELESS INJECTOR**
NADELLOSER INJEKTOR
INJECTEUR SANS AIGUILLE

(30) Priority: 22.12.2016 EP 16206086
(43) Date of publication of application: 30.10.2019
(73) Proprietor: Intervet International B.V., 5831 AN Boxmeer (NL)
(72) Inventor: FACHINGER, Vicky, 5831 AN Boxmeer (NL); COX, Erik, 5831 AN Boxmeer (NL)
(74) Representative: Intervet International B.V.
(86) International application number: PCT/EP2017/084031
(87) International publication number: WO 2018/115249

(56) References cited:
- WO-A1-03/103751
- WO-A1-2014/004462
- DE-U1- 20 016 079
- US-A1- 2002 065 483

## Description

### Field of the invention

The invention relates to a needle-less injector having a housing comprising two or more separate chambers defined within said injector for containing liquid to be injected wherein each chamber comprises a liquid outlet positioned at the front end of the injector and a dispensing member in contact with the liquid in said chamber and movable in a first direction to reduce the volume of said chambers to cause the liquid contained therein to be expelled through said liquid outlet.

### Background

Needle-free injection devices are known in the art for administering medicinal products to animals and humans. Instead of using a hypodermic needle, the needle-free injection devices make use of a narrow jet of a high-pressure fluid that is injected through the skin.

One typical application example of the needle-free injection devices is mass vaccinations in animals. The needle-free injection device is capable of delivering a target molecule at a variety of tissue depths ranging from the dermis to the muscle, depending on the force generated by the injector (Mitragotri S., Nat Rev Drug Discov. 2006; 5:543-548; Schramm-Baxter J., et al., J. Control Release, 2004; 97:527-535).

Known needle-free injection devices comprise a housing inside of which a chamber is formed for containing a medicinal product to be injected through a dispensing outlet out of the chamber. A dispensing mechanism is also provided for performing the injection of the medicinal product.

One example of a needle-free injection device is disclosed in document WO03103751. The needle-free injection device disclosed in this document comprises a chamber adapted for containing a product to be injected out of the chamber through a discharge nozzle. The chamber is connected through a supply line to a reservoir containing the product to be injected. The device includes a dispensing mechanism comprising a powered cam adapted for displacing a piston that is arranged in the chamber against a spring.

The device in WO03103751 comprises one chamber and one discharge nozzle as well as one dispensing mechanism. When more than one vaccine are to be injected, more than one device of WO03103751 need to be used or the vaccine composition needs to be exchanged for another which requires cleaning of the injection device. In some cases combination vaccine or poly-valent vaccines, comprising two or more different antigens may be used. Unfortunately, some antigens may not go well together and require separate vaccination on distinct injections sites to avoid interference of the antigens. For example, it was shown that simultaneous but independent delivery of four different dengue monovalent vaccines, i.e. multi-monovalent delivery, to the dermal layers provides all four monovalent vaccine viruses equal opportunity to replicate and elicit an immune response thus avoiding the interference observed when delivered in a single tetravalent formulation. US2002/0065483 and DE20016079U also describe needle-less injection devices for the simultaneous but independent administration of multiple substances/doses.

It is therefore desirable to provide a needle free injection device that delivers simultaneously compositions to discrete areas of the intradermal layers of skin. Preferably the compositions do not interfere with each other in the intradermal layers of skin.

Surprisingly it was found that this object can be met, and consequently one or more disadvantages of the prior art can be overcome, by providing a needle-less injector comprising two or more separate chambers within said injector for containing liquid to be injected wherein each chamber comprises a liquid outlet positioned at the front end of the injector; and comprises a dispensing member in contact with the liquid in said chambers to cause the liquid contained therein to be expelled through said liquid outlet. It was found that the distance between the liquid outlet for each of said chambers is at least 20 mm. It was found that when the distance between the liquid outlets is under 20 mm, local reactions in the skin occur whereas this is significantly less or even absent when the distance between the liquid outlets is at least 20 mm, whereas the device is still easy to be handled by one person.

WO2014/004462 discloses a multiple drug delivery device capable of using one or more vaccine cartridges having microneedles. The device may be used to perform multiple vaccine deliveries to the intradermal layer. The vaccine or drug is delivered via microneedles and is thus not needle-less. In addition, the cartridges are spaced at a distance of less than 1 cm from adjacent drug cartridges.

US2006/011663 discloses a needle-less injector comprising a plurality of nozzles. The nozzles are connected to a single interior cavity which is connected to a single ampule. The two nozzles are 0.173 inch apart. The aim is to deliver smaller dosages through the plurality of nozzles compared to the same total dosage delivered through a single nozzle ampule. The configuration allows the multi-nozzle fluid handling component to be used in higher dosage application, such as a 1 ml dosage application without delivery a large bolus to a single injection site.

### Summary of the invention

Therefore in a one aspect the invention relates to a needle-less injector having a housing wherein the housing comprises two or more separate chambers which are defined within said injector for containing liquid to be injected. Each chamber comprises a liquid outlet and a dispensing member. The liquid outlets are positioned at the front end of the injector. The dispensing member is in contact with the liquid in said chambers and is movable in a first direction to reduce the volume of said chamber to cause the liquid contained therein to be expelled through said liquid outlet. The needleless injector further comprising a drive means for actuating said injector. The distance between the liquid outlet for each of said chamber is at least 20 mm. It was found that when the liquid outlets of each chamber are at least 20 mm apart the liquid of each chamber is administered to the subject in discrete positions and no interference is observed. It was also found that if the liquid outlets of each chamber are at least 20 mm apart less or even no local reactions occur in the subject. Suitably the distance between the liquid outlet for each of said chamber is at least 25 mm, at least 27 mm, at least 29 mm, at least 32 mm, at least 35 mm, at least 40 mm, at least 45 mm or even at least 50 mm.

As indicated above it was surprising that is was possible to deliver two different liquids to a subject in discrete positions so that interference of the liquids and local reactions are a minimum, where at the same time the two separate chambers are able to be placed in a housing of an injection device and where it is still possible to handle this injection device without much effort. It was found that if the distance between the liquid outlet for each of said chamber is less than 100 mm it is still possible to use the injection device without much effort by a skilled person. Therefore preferably the distance between the liquid outlet for each of said chamber is less than 100 mm, less than 95 mm, less than 90 mm, less than 85 mm, less than 80 mm, less than 75 mm, less than 70 mm, less than 65 mm, or less than 60 mm.

Any type of needleless injector comprising two or more chambers for holding liquids and each chamber comprising a liquid outlet from which the liquid is expelled may be used in accordance with the invention and embodiments thereof as long as the distance between the liquid outlets of the chambers are as indicated.

A suitable needle-less injector that may be used in the present invention and embodiments thereof is an injector wherein the dispensing member is a spring-loaded piston movable in said chamber by the spring in the first direction to a preferred position at which the volume of said chamber is at a minimum and the spring is nearly unloaded, and which piston is movable in a second direction opposite to the first direction by actuation of the drive means whilst counteracting a force from the spring and moving the piston to a non-preferred position at which the spring is loaded. Suitably the piston is fixed to a movable member having a cam follower positioned on said member, and that the drive means is connected to a rotatable cam having a highest point and a lowermost point immediately following said highest point, which cam cooperates with said cam follower, so as to cause that rotation of the cam is converted into longitudinal movement of the member and the piston that is fixed to said member.

The injector of the present invention and embodiments thereof comprises at least two chambers; however it is advantageous if the dispensing of the liquid is actuated by a single measure such as switch or trigger. In such a case the person using the injector only needs to actuate the injector once to be able to administer two or more different liquids simultaneously. Therefore a needle-less injector according to the invention and embodiments is provided wherein the dispensing members are operated by a single trigger. Suitably the needle-less injector according to invention and/or embodiments comprises a single drive means for actuating said injector.

Advantageously the injector is able to be provided with a cartridge or container or vial containing a liquid to be injected so that the device can be used multiple times with different liquids. Preferably the injector is arranged to be able to accommodate a cartridge or container or vial containing a liquid and comprises a liquid inlet into the chamber. Therefore a needle-less injector according to the invention and embodiments is provided wherein the chambers have each a liquid inlet which is arranged to allow liquid to enter into the chamber when desired. Desirably the injector comprises supply lines that are able to conduct fluid from the container, or cartridge or vial containing a liquid to the liquid inlets. Therefore a needle-less injector according to the invention and embodiments is provided wherein for each chamber a supply-line is provided for letting in liquid into the chamber.

Suitably one may desire a safety mechanism so that the injector may only expel fluid when there is fluid present in the injector to avoid an 'empty' activation which may cause harm to the injector and to the person handling the injection. Suitable the needleless injector comprises a sensor that is able to detect liquid in the supply-line. Suitably, the needleless injector comprises one or more sensors that are able to detect liquid in the supply-line for each of said chambers. Suitably the operation of the drive means is dependent on the sensor(s), e.g via a switching means or blocking means.

It may also be advantageous to have the injector only be able to expel fluids when the injector is pressed to the skin of a subject. Such an action may suitably be performed by a pressure sensor, for example near the nozzles or liquid outlets. Suitably the pressure sensor will only allow to expel fluids when the injector is pressed with enough pressure to the skin of the subject and/or when in the correct position. Suitably the pressure sensor is connected to a switching means which controls the drive means and/or the power source. Therefore a needle-less injector according to the invention and embodiments is provided wherein the injector comprises a pressure sensor in order to sense axial pressure, which pressure sensor is connected to a first switching means within the housing which is connected to the drive means and a power source for said drive means, and which first switching means is capable to establish contact between the drive means for actuating said injector and the power source when the pressure sensor is actuated by a selected amount of axial pressure. Suitably, for each liquid outlet of each chamber a pressure sensor is provided to ensure that each outlet is positioned correctly before injection is actuated. However also a single pressure sensor is envisioned that is capable of sensing the correct position of the injector to the skin of the subject.

It may also be preferred that there is a trigger switch which can be controlled by the person using the injector so that the person can control whether fluid is injected or not. Suitably the trigger switch is connected to a switching means which controls the drive means and/or the power source. Therefore a needle-less injector according to the invention and/or embodiments is provided which comprises a trigger switch, which trigger switch is connected to a second switching means within the housing which is connected to the drive means and a power source for said drive means characterized in that in the actuated state of the trigger switch the second switching means is enabled to establish contact between the power source and the drive means for actuating the injector and which second switching means is capable to establish contact between the drive means for actuating said injector and the power source when the trigger switch is actuated by a selected amount of pressure on the trigger switch.

### Detailed description

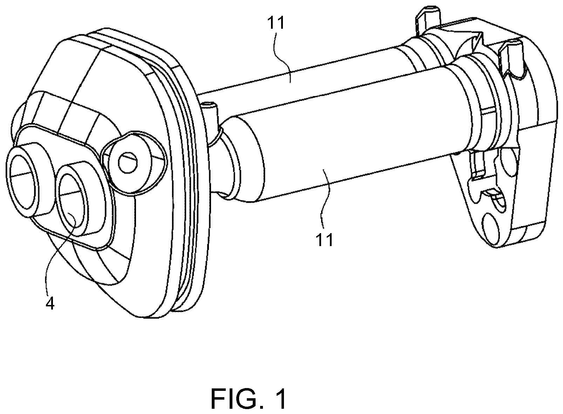

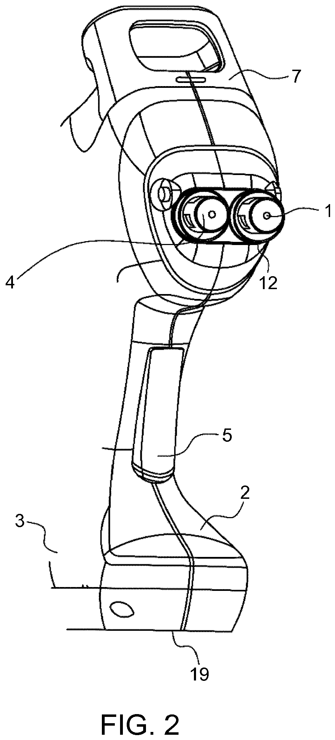

The present invention may suitably be described by figure 1. In here the two separate chambers (11) are indicated. Figure 2 shows then the liquid outlets (1), the housing (2, 3). The distance between the liquid outlets is the distance between the small openings where the liquid is to be expelled (1). This distance is at least 20 mm, preferably at least 22 mm, more preferably at least 26 mm, more preferably at leat 28 mm, more preferably at least 30 mm, more preferably at least 34 mm, more preferably at least 36 mm, more preferably at least 38 mm, more preferably at least 42 mm, more preferably at least 44 mm, more preferably at least 46 mm, more preferably at least 48mm.

It was found that if the distance between the liquid outlet for each of said chambers is less than 100 mm, it is still possible to use the injection device without much effort by a skilled person. Therefore preferably the distance between the liquid outlet for each of said chambers is less than 100 mm, less than 98 mm, less than 96 mm, less than 94 mm, less than 92 mm, less than 88 mm, less than 84 mm, less than 78 mm, less than 74 mm, less than 72 mm, less than 68 mm, less than 64 mm, or less than 62 mm.

Each chamber comprises a liquid outlet and a dispensing member. The dispensing member is in contact with the liquid in said chambers and is movable in a first direction to reduce the volume of said chamber to cause the liquid contained therein to be expelled through said liquid outlet. Suitably the dispensing member is a spring-loaded piston movable in said chamber by the spring in the first direction to a preferred position at which the volume of said chamber is at a minimum and the spring is nearly unloaded, and which piston is movable in a second direction opposite to the first direction by actuation of the drive means whilst counteracting a force from the spring and moving the piston to a non-preferred position at which the spring is loaded. The advantage of such dispensing member is that a separate impacting member to cause movement of the dispensing member is avoided. A particularly suitable construction of the needle-less injector according to the invention is characterized in that the piston is fixed to a movable member having a cam follower positioned on said member, and that the drive means is connected to a rotatable cam having a highest point and a lowermost point immediately following said highest point, which cam co-operates with said cam follower, so as to cause that rotation of the cam is converted into longitudinal movement of the member and the piston that is fixed to said member.

Suitably at least part of the liquid inlet of the chamber is formed by the front end of the piston. This construction offers some advantages which shall become apparent from the further discussion below. Further at least part of the liquid inlet of the chamber may be formed by a central bore extending through at least part of the piston, which central bore has an outlet to the chamber. This construction offers some further advantages which shall become apparent from the further discussion below. It is advantageous that near the non- preferred position of the piston the central bore is in open fluid communication with a supply-line for the fluid.

The benefits of the just-mentioned construction are completely attained when the supply-line has an outlet adjacent to which sealing organs are provided, that co-operate with the piston and that the piston is provided with at least one essentially radial channel that extends to the bore within the piston and which channel has an opening at the piston's circumference that is in open fluid communication with the outlet of the supply line only when the piston is near the non-preferred position. Suitably the sealing organs are O-rings, and the piston is moveable through said O-rings.

Entirely depending on the position that the piston assumes with respect to the opening of the supply-line, the chambers then can be filled with liquid to be used for injecting purposes. Whilst retracting the piston from the preferred position to the non-preferred position the liquid outlet of each of the chambers may be closed by the action of a non-return valve.

Consequently the retraction of the piston causes under-pressure in the chambers. At the moment the piston assumes or is near the non-preferred position the liquid for injecting purposes flows from the outlet of the supply-line through the piston's radial channel and bore to the chamber under the influence of the under-pressure that is present in each of said chamber.

Another advantage of the above indicated constructions is the following: When the piston is in the non-preferred position and the needle-less injector is ready for operation so as to cause the liquid to be expelled through the injector's liquid outlets, the piston can initially be accelerated in order to reduce the volume of the chamber in which the liquid is contained, which acceleration can occur without much loss or friction. The access amount of liquid in each of the chambers which would otherwise restrict the acceleration of the piston can initially leave each of the chamber through the piston's central bore and radial channel by means of its open fluid communication with the supply line for the fluid. This can continue up to the moment that the piston has left the non-preferred position to such extend that the open fluid communication of the piston's radial channel with the outlet of the supply-line is lost.

Suitably the supply- line is provided with a sensor for detecting the presence of liquid for injecting purposes, whilst the operation of the drive means is dependent on the sensor. It may further be desirable that the drive means are enabled to cause the motor to be enabled when the sensor detects liquid for injecting purposes. When that happens the moveable member and the piston connected thereto progressively diminish the volume of each of said chambers. Initially the piston then accelerates quickly due to the access amount of liquid in the chamber being able to leave said chamber through the central bore, the radial channel connected thereto and from there through the outlet back into the supply line. With the continued motion of the piston the radial channel moves past the left O-ring and closes off the open fluid communication between the central bore and the outlet of the supply line resulting eventually in expelling the liquid contained in the chamber whilst passing the non-return valve in order to effectuate an injection with that liquid. Thereafter the motor may retract the piston from its preferred position arrived at when the volume of chamber is at its minimum to return to the starting position in order to repeat the injecting operation.

The sensor may e.g. be a combination of a light emitting diode (LED) and a light-sensitive detector opposite the LED, placed over the supply-line. The supply-line is preferably made of a transparent material such as Teflon.

If no liquid or a colorless cleaning liquid such as water is present in the supply-line, the light of the LED will be detected by the light-sensitive sensor. If however a liquid for injection purposes passes the sensor, less or no light of the LED will be detected by the sensor. This is due to the fact that liquid for injection purposes is by nature practically always opaque.

The operation of the needle-less injector according to the invention may be identical to the manner of operation of the needle-less injector according to the prior art. For instance this operation may be dependent on the front end portion being placed under pressure against the epidermis of an animal. A suitable dispensing member is described In WO03103751 or WO9813085.

In some embodiments the injector comprises a trigger switch. In the actuated state of the trigger switch switching means are enabled to establish contact between a power source and the drive means for actuating the injector to cause the liquid to be expelled from the chamber through the said liquid outlet, and that the switching means disable further contact between the power source and the drive means until at least the trigger switch is no longer actuated and brings the injector back to the unloaded state.

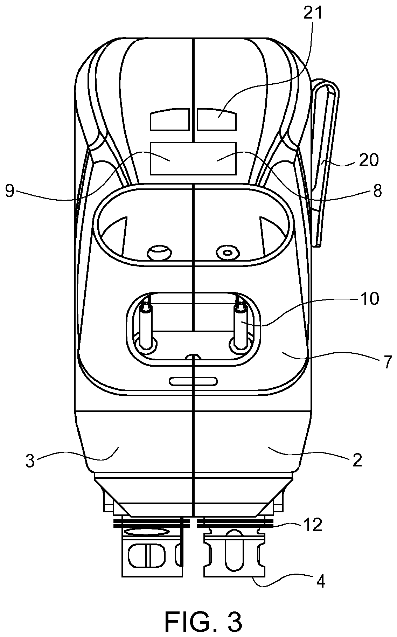

The injector may also have a pressure sensor. Advantageously the pressure sensor is a front portion which is movable with respect to the housing by the selected amount of axial pressure from an unloaded to a loaded position. An example is shown in figure 3 showing a top view of an injector according to the invention and embodiments thereof. In figure 3 the pressure sensor (12) is positioned in the front of injector and is movable with respect to the housing by applying pressure, e.g. when the injector is pressed against the skin of the animal to be injected. The pressure sensor may be electronic or a simple mechanical solution such as a rod extending from the housing; a reliable and preferred mechanical solution is however characterized in that the pressure sensor is a front portion which is movable with respect to the housing by the selected amount of axial pressure from an unloaded to a loaded position.

In an example the injector may be described as follows: The injector comprises a housing (2, 3) to which a movable front portion 12 is attached which can be moved in the direction the housing when loaded due to placement against the epidermis of a human, animal or plant. A spring via rod, and a spring via pin urge the front portion 12 to assume the unloaded position distant from the housing (2,3). This position is shown in the drawing of figure 3. The front end supports a cylinder with a chamber 11 for the liquid, in which cylinder a piston may be sealingly located. The piston is preferably hollow, but closed at both ends, in the case of the right hand end by a hard cap. The cylinder may be connected via a non-return valve, biased to its closed position by a compression spring, and a tube to a reservoir containing a liquid to be injected. The reservoir advantageously has an air inlet to permit air to enter the bottle as the liquid is dispensed therefrom. A discharge nozzle 4 is sealinglv connected to the chamber 11 within the cylinder, and a non-return valve, biased to its closed position by a compression spring, prevents air being drawn into the cylinder during the induction stroke.

The piston may be loosely located within a hole in the end of a connecting rod, so that it may move freely in a longitudinal direction. A pin may be fixed to the piston, the pin extending radially therefrom on opposite sides thereof. The pin slides in a slot in a connecting rod. The connecting rod is slidingly located in bearings, and urged in the forward direction by a compression spring.

A motor-gearbox assembly may be positioned below the two chambers. The motor is described below as being electric, but could be of some other type, for example gas powered.

In a particular embodiment, in the semi-loaded position the volume of the chamber is near its maximum due to the fact that in this position the impacting member or piston largely is removed from the area of this chamber. In an alternative embodiment having the front portion in the unloaded position, the piston would largely fill the area of this chamber. When in this latter embodiment of the injector according to the invention the front portion is then moved towards the housing, a switch is activated. This puts switching means, for instance a logic circuit or a small microprocessor, into an enabled state to establish contact between a power source and the drive means. The operation of the injector further may require activating a trigger switch 5 providing an enabling input for the above-mentioned switching means. In an alternative embodiment, the switching means 5 could be enabled solely by a switch so as to establish contact between the power source and the drive means. For example the trigger switch brings the injector in a semi-loaded position until also the front portion is moved from the unloaded into its loaded position. In this latter situation, the injector will only expel the fluid if simultaneous actuation of the switch (by the trigger switch) and the front portion (by the sensor pressure) occurs.

The above-mentioned actuation of the drive means may effect moving of the rod or impacting member away from the piston or dispensing member against the biasing force provided by the spring. As the connecting rod retracts, the piston initially remains stationary, until the left-hand ends of the slots in the connecting rod are contacted by pins in the piston. The piston then travels with the connecting rod and draws injection liquid from a reservoir via the supply line into the chamber. This affects releasing of the rod or impacting member to permit it to travel towards and impact against the piston to cause the liquid which has been drawn into the chamber to be expelled therefrom through liquid outlet. After release of the liquid from the chamber through the liquid outlet, the switching means disable further contact between the power source and the drive means until at least the front portion has assumed the unloaded position again.

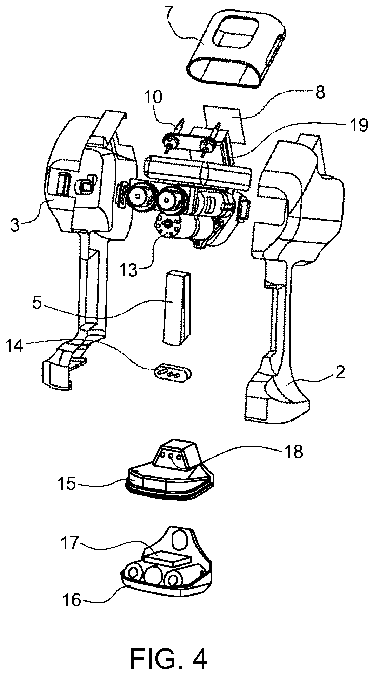

Suitably the injector of the present invention and/or embodiments thereof is adapted to inject the medicinal product through the skin transdermally, intradermally, subcutaneously or intramuscularly. A medicinal substance or product refers to any substance or combination of substances that can be used to prevent or treat a disorder, including diseases, i.e., to aid in preventing, ameliorating, treating or curing the disorder. Such a substance may for example be a chemical, pharmaceutical or biological compound, such as a natural or synthetic peptide or protein, a (poly-)saccharide or any other organic or inorganic molecule, a killed or a live micro-organism, such as bacteria, virus, fungus, phages, parasite, etc. The medicinal product may be supplied by a container, such as a bottle, or vial. Suitably the container is placed in the container receptacle (7). Figure 4 shows such a container receptacle adapted to hold two containers simultaneously. The supply needles (10) as indicated in figure 4 may penetrate the container to allow fluid from the container to flow via the supply-line to the chamber.

Notwithstanding the foregoing, the container may also contain cleaning products such as sanitization liquids or solutions, for example, benzyl alcohol, in order to decontaminate and clean the device before and after the injection sessions, e.g. the vaccination sessions.

The container receptacle may contain different types of containers. Examples of containers that can be received into the container receptacle are vials, flasks or the like which are well-known for those skilled in the art. Said containers for containing the medicinal products to be injected may be of different nature. For example they may be made of glass or plastic materials such as for example HDPE (High Density Polyethylene), LDPE (Low Density Polyethylene), PP (Polypropylene), PET (Polyethylene Terephthalate), etc.

As used herein, injection involves administering said medicinal product to an animal through the skin (i.e. transdermal route), and specifically by intradermal route. Intramuscular or subcutaneous administration may be also possible depending on the injection parameters (injection pressure, injection volume, diameter of the nozzle) which may be set in the device.

Reference signs related to drawings and placed in parentheses in a claim, are solely for attempting to increase the intelligibility of the claim, and shall not be construed as limiting the scope of the claim.

The invention will now be further described by the following, non-limiting, examples.

### Examples

### Example 1

A total of 60 piglets with antibodies (MDA) against PCV2 were allotted to 6 treatment groups: six groups of 10 piglets each. All groups were vaccinated needle-less when the piglets were approximately three weeks old. Piglets from groups 1 through 4 were vaccinated with 0, 1, 2 and 3 mm distance to the skin with a single dose of Porcilis PCV ID (0.2 ml). Piglets from groups 5 through 8 were vaccinated with Lawsonia Freeze Dried (LFD) mixed in Porcilis PCV ID (0.2 ml) 0, 1, 2, 3 cm apart from the administration site of Porcilis M Hyo ID ONCE (0.2 ml). All vaccines of group 1-8 were administered in the right side of the neck. Piglets from group 9 were vaccinated concurrently with Porcilis PCV ID mixed with LFD (0.2 ml) in the right side of the neck and Porcilis M Hyo ID ONCE (0.2 ml) in the left side of the neck. Group 10 was not vaccinated (control group).

All piglets were observed daily after vaccination for clinical signs. Local reactions were monitored by palpation every second day, starting on the day of vaccination until 28 days post vaccination. Serum samples were collected from all animals on the day of vaccination as well as 3 and 4 weeks after vaccination. Samples were tested for antibodies against PCV2 and were compared to each other.

Following vaccination the severity of local reactions with animals vaccinated only with Porcilis PCV ID (group 1-4) with regulated distance from the skin were all below 0.7 cm with a maximum local reaction of 4 cm (group 3). For the groups where Porcilis PCV ID was mixed with LFD and concurrently vaccinated with Porcilis M Hyo ID ONCE (group 5-8) was the highest in group 6 (average maximum size 2.1 cm). The lowest local reactions were in the group that was vaccinated concurrently (group 9). The average maximum local reactions were comparable between the other test groups (between 1.2 and 1.6 cm)

At the time of vaccination (SDO) the average PCV2 total Ig antibody titer was moderate in all groups (average 8.1 log₂) and all animals were negative for PCV2 IgM antibodies.

Following vaccination the average PCV2 total antibody titer remained similar in all the vaccinated groups and decreased in the control group. Three and four weeks post vaccination (SD21 and SD28) groups 2 (PCV ID 1mm) and 4 (PCV ID 3mm) had a slightly weaker IgM response than all other vaccinated groups. The rest of the vaccinated groups had a 90-100% IgM response.

At the start of the study 30-50% of all animals were positive for antibodies against M Hyo. Following vaccination at 3wpv 0% of the animals were positive against M Hyo. At 4wpv 10% of the animals from group 5 and 7 were positive and 20% of the animals from group 9 were positive for antibodies against M Hyo.

At the start of the study all animals were negative for antibodies against Lawsonia. During the course of the study the percentage of positive animals in the groups with the Twin injector (5, 6, 7 and 8) increased to 50% to 80% at the end of the study. The percentage of positive animals in the group that was vaccinated on both sides of the neck (group 9) were less (30%) than the Twin injector groups. None of the animals in the control group had a Lawsonia serological response throughout the study.

**Table 1: vaccination scheme**

| No. of animals | Group | First Vaccine | Application* | Second Vaccine | Application* | Distance |
|---|---|---|---|---|---|---|
| 10 | 1 | Porcilis PCV ID | 0.2 ml ID R | - | - | 0mm distance from skin |
| 10 | 2 | Porcilis PCV ID | 0.2 ml ID R | - | - | 1mm distance from skin |
| 10 | 3 | Porcilis PCV ID | 0.2 ml ID R | - | - | 2mm distance from skin |
| 10 | 4 | Porcilis PCV ID | 0.2 ml ID R | - | - | 3mm distance from skin |
| 10 | 5 | Porcilis PCV ID+LFD | 0.2 ml ID R | Porcilis M Hyo ID Once | 0.2 ml ID R | 0cm right (same injection site) |
| 10 | 6 | Porcilis PCV ID+LFD | 0.2 ml ID R | Porcilis M Hyo ID Once | 0.2 ml ID R | 1cm apart# |
| 10 | 7 | Porcilis PCV ID+LFD | 0.2 ml ID R | Porcilis M Hyo ID Once | 0.2 ml ID R | 2cm apart# |
| 10 | 8 | Porcilis PCV ID+LFD | 0.2 ml ID R | Porcilis M Hyo ID Once | 0.2 ml ID R | 3cm apart# |
| 10 | 9 | Porcilis PCV ID+LFD | 0.2 ml ID R | Porcilis M Hyo ID Once | 0.2 ml ID L | - |
| 10 | 10 | None | - | - | - | - |

| | | | | | | |
|---|---|---|---|---|---|---|
| *ID Intradermal; R (right side of neck); L (left side of neck) # indicates distance between the outlets | | | | | | |

### Experimental procedures

### Daily observation

All pigs were observed daily for clinical signs of disease. On the day of vaccination animals were observed before as well as 4 hours after vaccination. Observations consisted of systemic reactions such as loss of appetite, reluctance to move, tendency to lie down, listlessness or drowsiness, shivering, bristling, oedema (especially around the eyes), vomiting and diarrhoea and dyspnoea.

### Palpation

All study pigs were palpated for the occurrence of local reactions at the injection site. Palpations were done in the right side of the neck or for group 1 through 8 and 10 and in the right and left side of the neck for group 9 every second day from the 2nd day post vaccination until 28 days post vaccination. The diameter and a description of the kind of reaction such as firmness (hard-soft), colour (red-blue), extension (diffuse-focal), temperature (warm-cold) and painfulness was recorded. In case of two local reactions apart from injection site, the aspects of both local reactions were recorded. For determination of the diameter a ruler was used.

### Sampling of blood

Blood samples were collected on the day of vaccination, and 3 and 4 weeks later. This was done from all pigs individually.

### Serology

Total PCV2 antibody ELISA Sera were tested for antibodies against PCV2.

In brief, serially diluted serum samples were incubated on microtiter plates coated with baculovirus expressed PCV2 ORF2 antigen. After removing the sera, all wells were incubated with a fixed amount of biotin-labeled PCV2-specific monoclonal antibody (MoAb). Bound MoAb is then incubated with peroxidase-conjugated streptavidin followed by chromophoric detection.

Results were expressed as log₂ titers.

### PCV2 IgM antibody ELISA

To determine the PCV2 specific IgM serological response a qualitative approach was used in which the S/P ratio was determined.

Sera of 0, 3 and 4 wpv were tested for IgM antibodies against PCV2 according to the following procedure. In brief, 25x diluted serum samples were incubated in two-fold on microtiter plates coated with IgM antibody. After removing the sera, all wells were incubated with baculovirus expressed PCV2 ORF2 antigen. Then all wells were incubated with a fixed amount of biotin-labelled PCV2 specific monoclonal antibody. Bound MoAb was finally incubated with peroxidase- conjugated streptavidin followed by chromophoric detection. Results were determined based on the S/P ratio relative to the cut-off and were expressed as positive or negative.

### Lawsonia antibody ELISA

Relevant sera were tested with a commercial test (ELISA-LAW-BioScreen). In short, Lawsonia antigen was coated to microtitre plates. After coating the plates were washed and serial three-fold dilutions of sera were made. After incubation and subsequent washing the bound antibodies were quantified by using anti-pig conjugate and TMB as substrate.

### MHyo antibody ELISA

Relevant sera were analysed with a commercial test (ELISA-MH-IDEXX) according to the manufacturer's instructions.

### Evaluation / interpretation of results

Groups one through nine were compared to group ten and to each other to determine differences in safety and efficacy of the vaccines after intradermal application.

At study day 12 (SD12) animal 66 (group 7) was treated with depocilline (Procaine penicillin) and after two days animal was healthy again. At SD19 animal 135 (group 4) was found dead. At necropsy severe post mortem decay was present; cause of death could not be established.

None of the other animals showed any sign of disease.

### Local reactions

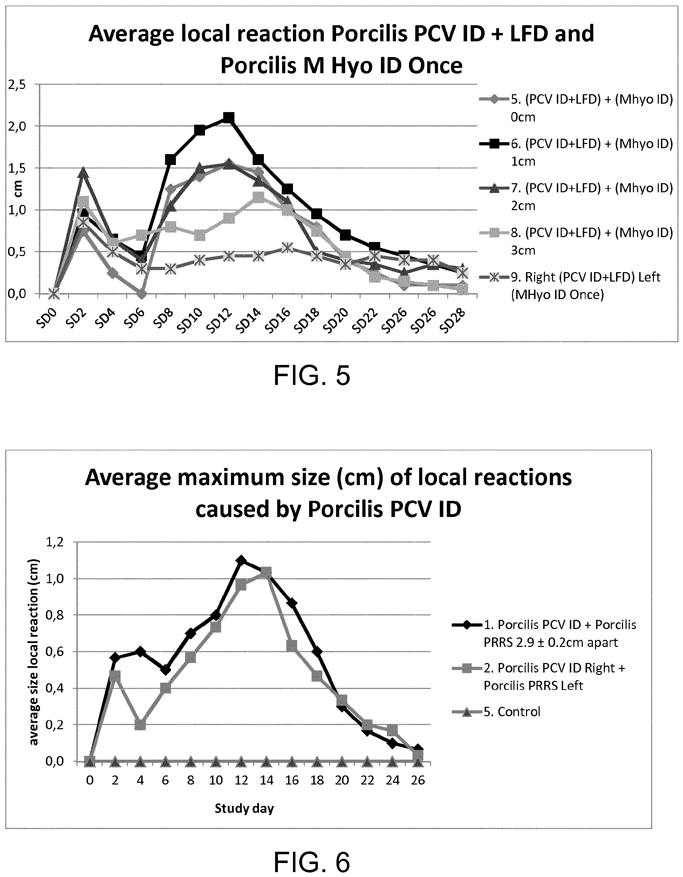

Average local reactions over the time for the groups with the twin injector are summarized in Figure 5.

Following vaccination at different distances from the skin average local reactions were low (max 0.7cm) in all groups with a maximum local reaction of 4 cm (animal 125, group 3) and 60-100% of the animals with a local reaction. A distance of 0 and 2mm resulted in the largest average local reactions. The maximum local reaction was the largest in group 3 (4 cm).

For the groups vaccinated concurrently, the size of the local reactions was summed up in case of two local reactions to make a comparison between groups. Following concurrent vaccination the severity of local reactions in the groups where Porcilis PCV ID was mixed with LFD and concurrently vaccinated with Porcilis M Hyo ID ONCE was the highest in the group with 1cm distance between vaccination sites (group 6 average maximum size 2.1 cm). The smallest local reactions were in the group that was vaccinated on both sides of the neck (group 9). The average maximum local reactions were comparable between the other test groups (between 1.2 and 1.6 cm).

### PCV2 serology

Results of the PCV2 serology are summarized in Table 2.

At the time of vaccination (SDO) the average PCV2 total Ig antibody titre was moderate in all groups (average 8.1 log₂) and all animals were negative for PCV2 IgM antibodies.

Following vaccination the average PCV2 total antibody titre remained similar in all the vaccinated groups and decreased in the control group. Three and four weeks post vaccination (3 and 4 wpv) groups 2 (PCV ID 1mm) and 4 (PCV ID 3mm) had a slightly weaker IgM response than all other vaccinated groups. The rest of the vaccinated groups had a 90-100% IgM response.

**Table 2: Average PCV2 specific serological response following vaccination**

| Group no. | Group | Ab ELISA (log2) | | | IgM Response (% positive animals) | | |
|---|---|---|---|---|---|---|---|
| | | 0wpv | 3wpv | 4wpv | 0wpv | 3wpv | 4wpv |
| 1 | PCV ID 0mm | 7.6 | 7.2 | 7.6 | 0.0 | 90.0 | 100.0 |
| 2 | PCV ID 1mm | 8.0 | 6.4 | 6.4 | 0.0 | 60.0 | 60.0 |
| 3 | PCV ID 2mm | 8.3 | 6.9 | 7.3 | 0.0 | 100.0 | 100.0 |
| 4 | PCV ID 3mm | 8.4 | 6.8 | 6.6 | 0.0 | 77.8 | 66.7 |
| 5 | (PCV ID+LFD) + M Hyo ID 0cm | 8.0 | 6.9 | 7.4 | 0.0 | 90.0 | 100.0 |
| 6 | (PCV ID+LFD) + M Hyo ID 1cm | 8.5 | 7.2 | 7.6 | 0.0 | 100.0 | 100.0 |
| 7 | (PCV ID+LFD) + M Hyo ID 2cm | 8.1 | 7.0 | 6.9 | 0.0 | 80.0 | 100.0 |
| 8 | (PCV ID+LFD) + M Hyo ID 3cm | 8.6 | 7.3 | 7.5 | 0.0 | 80.0 | 90.0 |
| 9 | (PCV ID+LFD) R + M Hyo ID L | 7.4 | 6.6 | 6.0 | 0.0 | 90.0 | 90.0 |
| 10 | Control | 7.7 | 5.8 | 5.5 | 0.0 | 0.0 | 0.0 |

### Example 2:

A total of 65 piglets with moderate antibodies (MDA) against PCV2 were allotted to 4 treatment groups of 15 piglets each. The control group contained 5 piglets and was not vaccinated. All groups were vaccinated intradermally when the piglets were approximately five weeks old.

Piglets were vaccinated in the neck with Porcilis PCV ID and Porcilis PRRS or Porcilis M Hyo ID ONCE and Porcilis PRRS either 2.9cm ± 0.2cm apart from each administration site or on the left and the right side of the piglet.

All piglets were observed daily after vaccination for clinical signs. Local reactions were monitored by palpation every second day, starting on the day of vaccination until 26 days post vaccination. On SD10 and SD15 pictures were taken from the local reaction of each animal. Serum samples were collected from all animals on SD0, SD15, SD22, and SD28 after vaccination. Samples were tested for antibodies against PCV2, PRRSV and Mhyo and were compared to each other.

Following vaccination the severity of local reactions varied between de different treatment groups. The average maximum size varied from 0.7cm to 1.5cm with a maximum size of 2.0cm. All the animals that were vaccinated showed a local reaction. When vaccinating Porcilis PCV ID or Porcilis M Hyo ID ONCE at 2.9 ± 0.2cm from Porcilis PRRS, the local reaction of Porcilis PRRS and Porcilis M Hyo ID ONCE was more severe in comparison when vaccinating on both sides.

At the time of vaccination (SDO) the average PCV2 total Ig antibody titre was relatively high in all groups (average 7.2 log₂) and all animals were negative for PCV2 IgM antibodies, except one animal from group 4, this animal had an IgM serological response with a S/P ratio slightly above the threshold.

Following vaccination the average PCV2 total antibody titre remained similar in the groups vaccinated with Porcilis PCV ID and decreased in the other vaccinated groups and the control group. At SD14 100% of the animals in the groups vaccinated with Porcilis PCV ID had an IgM serological response. At the end of the study (SD28) the PCV2 specific total antibody response in the groups vaccinated with Porcilis PCV ID (groups 1-2) was considerably higher than the control group.

At the time of vaccination (SDO) all groups were negative for PRRSV antibody titres. The percentage of animals that were positive for PRRSV specific antibodies increased to 100% towards the time point of the end of the study (SD28). No differences were seen between the four Porcilis PRRS vaccinated groups.

From this study the following can be concluded:
- There is no difference between the local reactions and serological response of Porcilis PCV ID, whether it is vaccinated 2.9cm ± 0.2cm apart from Porcilis PRRS or vaccinated on the other side of the neck.
- The local reactions of Porcilis M Hyo ID ONCE vaccinated left and Porcilis PRRS right endured longer than the group that was vaccinated 2.9cm ± 0.2cm apart from Porcilis PRRS.
- The local reactions caused by vaccination with Porcilis PRRS increased slightly when vaccinating in close proximity of Porcilis PCV ID or Porcilis M Hyo ID ONCE.
- There was no negative effect on the PRRS serological efficacy between all the groups when vaccinated 2.9cm ± 0.2cm apart or on both sides of the piglet.

### Dosage and administration

Vaccinations were done by the intradermal route, 0.2ml, on the right or left side of the neck. Vaccinations were recorded on standard forms.

**Table 3: Vaccination scheme**

| No. of anima Is | Group | Vaccine | Application* | Vaccine | Application* | Distance |
|---|---|---|---|---|---|---|
| 15 | 1 | Porcilis PCV ID | 0.2 ml ID R | Porcilis PRRS | 0.2ml ID R | 2.9cm ± 0.2cm apart# |
| 15 | 2 | Porcilis PCV ID | 0.2 ml ID R | Porcilis PRRS | 0.2ml ID L | - |
| 15 | 3 | Porcilis M Hyo ID ONCE | 0.2 ml ID R | Porcilis PRRS | 0.2ml ID R | 2.9cm ± 0.2cm apart# |
| 15 | 4 | Porcilis M Hyo ID ONCE | 0.2 ml ID R | Porcilis PRRS | 0.2ml ID L | - |
| 5 | 5 | - | - | - | - | - |

| | | | | | | |
|---|---|---|---|---|---|---|
| *ID Intradermal; R (right side of neck); L (left side of neck) # indicates distance between outlets. Blood samples were taken on the day of vaccination and 15, 22 and 28 days after vaccination. | | | | | | |

### RESULTS

### Local reactions

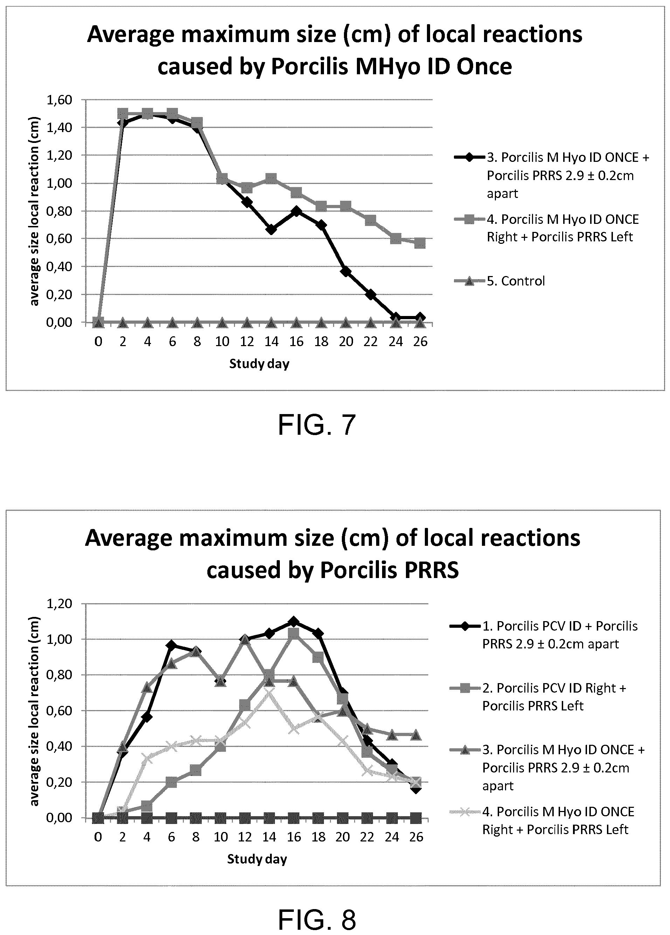

Local reactions are summarized in Figure 6, 7 and 8. In case of possible two local reactions per animal of different vaccines the local reactions were divided into three different graphs. All animals that were vaccinated showed local reactions. The control group showed no local reactions.

### Local reactions Porcilis PCV ID (with Porcilis PRRS 2.9cm ± 0.2cm apart or both sides of the neck)

Following vaccination the severity of local reactions (average maximum size, percentage of animals with local reactions) was comparable between the two groups that were vaccinated with Porcilis PCV ID (group 1 and 2). The average maximum size for group 1 was 1.1cm. At SD26 13% of the animals still had a small local reaction. The average maximum size for group 2 was 1.0cm. At the end of the study 7% (1 animal) still had a small local reaction.

### Local reactions Porcilis M Hyo ID ONCE (with Porcilis PRRS 2.9cm ± 0.2cm apart or on both sides of the neck)

Following vaccination the severity of local reactions (average maximum size, percentage of animals with local reactions) was comparable between the two groups that were vaccinated with Porcilis M Hyo ID ONCE (group 3 and 4) until SD12. From SD12 onwards the local reactions of group 4 stayed at a higher average maximum size until end of study. The average maximum size for group 3 was 1.5cm. At SD 24 all local reactions had waned. The average maximum size for group 4 was 1.5cm. At the end of the study 87% of the animals still had small (average 0.6cm) local reactions.

### Local reactions Porcilis PRRS (with Porcilis PCV ID or Porcilis M Hyo ID ONCE 2.9cm ± 0.2cm apart or concurrent)

Following vaccination the severity of local reactions due to Porcilis PRRS (average maximum size, percentage of animals with local reactions) was comparable between the groups that were vaccinated 2.9cm ± 0.2cm apart from Porcilis PCV ID or Porcilis M Hyo ID ONCE (group 1 average maximum size 1.1cm and group 3 average maximum size 1.0cm) and the group where Porcilis PCV ID was vaccinated on the other side of the neck compared to Porcilis PRRS (group 2 average maximum size 1.0cm). The local reactions were slightly lower in the group that was vaccinated on both sides of the neck with Porcilis M Hyo ID ONCE and Porcilis PRRS (average maximum size 0.7cm).

### PCV2 serology

Individual results of the PCV2 serology are shown in in Table 4.

**Table 4: Average PCV2 specific serological response following vaccination.**

| | **Total Ab ELISA (log2)** | | | | **IgM response (% positive animals)** | | | |
|---|---|---|---|---|---|---|---|---|
| | **SD0** | **SD14** | **SD20** | **SD28** | **SD0** | **SD14** | **SD20** | **SD28** |
| 1. PCV ID + PRRS (2.9cm ± 0.2cm apart) | 6.9 | 6.8 | 8.2 | 8.9 | 0.0 | 100.0 | 93.3 | 100.0 |
| 2. PCV ID + PRRS (both sides) | 7.1 | 6.5 | 8.5 | 7.8 | 0.0 | 100.0 | 100.0 | 93.3 |
| 3. M Hyo ID + PRRS (2.9cm ± 0.2cm apart) | 7.2 | 5.6 | 4.8 | 3.9 | 0.0 | 6.7 | 13.3 | 0.0 |
| 4. M Hyo ID + PRRS (both sides) | 7.1 | 6.0 | 6.0 | 4.3 | 13.3 | 13.3 | 6.7 | 0.0 |
| 5. Control | 8.0 | 6.9 | 6.5 | 4.6 | 0.0 | 0.0 | 0.0 | 20.0 |

At the time of vaccination (SDO) the average PCV2 total Ig antibody titre was moderate in all groups (average 7.2 log2) and all animals were negative for PCV2 IgM antibodies, except one animal from group 4, this animal had an IgM serological response with a S/P ratio slightly above the threshold.

Following vaccination the average PCV2 total antibody titre increased in the groups vaccinated with Porcilis PCV ID and decreased in the other vaccinated groups and the control group. At SD14 100% of the animals in the groups vaccinated with Porcilis PCV ID had an IgM serological response. At the end of the study (SD28) the PCV2 specific total antibody response in the groups vaccinated with Porcilis PCV ID (groups 1-2) was considerably higher than the control group.

During the study, some animals from group 3 and 4 and in the control group were found to have a PCV2 specific IgM serological response with an S/P ratio slightly above the threshold, indicating a possible PCV2 field infection during the study.

### PRRSV serology

Individual results of the PCV2 serology are shown in Table 5.

At the time of vaccination (SDO) all groups were negative for PRRSV antibody titres. From SD14 onwards animals from group 1 through 4 started to seroconvert and the percentage of animals that were positive for PRRSV specific antibodies increased to 100% towards the end of the study (SD28). No differences were seen between the four Porcilis PRRS vaccinated groups.

**Table 5: Percentage PRRS serological response following vaccination.**

| | **SD0** | | **SD14** | | **SD20** | | **SD28** | |
|---|---|---|---|---|---|---|---|---|
| | S/P | % Pos/Neg | S/P | % Pos/Neg | S/P | % Pos/Neg | S/P | % Pos/Neg |
| 1. PCV ID + PRRS (2.9cm ± 0.2cm apart) | 0.0 | 0.0 | 1.1 | 80.0 | 1.8 | 100.0 | 2.1 | 100.0 |
| 2. PCV ID + PRRS (both sides) | 0.0 | 0.0 | 1.3 | 93.3 | 2.0 | 100.0 | 2.1 | 100.0 |
| 3. M Hyo ID ONCE + PRRS (2.9cm ± 0.2cm apart) | 0.0 | 0.0 | 1.4 | 100.0 | 1.9 | 100.0 | 2.1 | 100.0 |
| 4. M Hyo ID ONCE + PRRS (both sides) | 0.0 | 0.0 | 1.1 | 80.0 | 1.7 | 93.3 | 1.8 | 100.0 |
| 5. Control | 0.0 | 0.0 | 0.0 | 0.0 | 0.0 | 0.0 | 0.0 | 0.0 |

### Legend to the figures

Figure 1: Part of the inside of the injector showing the cylinders comprising the two chambers from which the fluid may be expelled through the fluid outlet.
Figure 2: View of the injector showing the two liquid outlets (1) in the nozzles (4), the trigger switch (5), housing (2,3) the receptacle for the containers (7), the pressure switch (12) and the battery holder (19).
Figure 3: View from above showing the housing (2,3), the nozzles (4), the pressure switch (12), the receptacle for the containers (7), the supply needles (10), the display (9) and the touch screen (8), buttons for controlling the menu (21), and a belt clip (20).
Figure 4: Schematic view of the injector showing the housing (2,3), the nozzles (4), the liquid outlets (1), the pressure switch (12), the receptacle for the containers (7), the supply needles (10), the display (9) and the touch screen (8), trigger switch (5), contact for the battery (14), motor (13), top shell (15) and bottom shell (16) for battery, switch for battery (17), contacts for battery (18).
Figure 5: Average local reactions over time for Porcilis PCV ID mixed with Lawsonia FD and concurrent with Porcilis M Hyo ID ONCE with different distance between vaccinations. (Local reactions were summed up in case of two visible local reactions).
Figure 6: Average maximum size of local reactions caused by Porcilis PCV ID
Figure 7: Average maximum size of local reactions caused by Porcilis MHyo ID ONCE
Figure 8: Average maximum size of local reactions caused by Poricilis PRRS

## Claims

1. A needle-less injector having a housing (2, 3) comprising:
two or more separate chambers (11) defined within said injector for containing liquid to be injected;
- each chamber comprising
- a liquid outlet (1) positioned at the front end of the injector; and
- a dispensing member in contact with the liquid in said chambers and movable in a first direction to reduce the volume of said chamber to cause the liquid contained therein to be expelled through said liquid outlet;
the needleless injector further comprising a drive means for actuating said injector,
**characterised in that** the distance between the liquid outlet for each of said chambers is at least 20 mm.

2. A needle-less injector according to claim 1 wherein the distance between the liquid outlet for each of said chambers is at least 29 mm.

3. A needle-less injector according to any one of claim 1 or 2 wherein the distance between the liquid outlet for each of said chambers is at least 32 mm.

4. A needle-less injector according to any one of claim 1 to 3 wherein the distance between the liquid outlet for each of said chambers is at most 100 mm.

5. A needle-less injector according to any one of claim 1 to 4 wherein the dispensing member is a spring-loaded piston movable in said chamber by the spring in the first direction to a preferred position at which the volume of said chamber is at a minimum and the spring is nearly unloaded, and which piston is movable in a second direction opposite to the first direction by actuation of the drive means whilst counteracting a force from the spring and moving the piston to a non-preferred position at which the spring is loaded.

6. A needle-less injector according to any one of claim 1 to 5 wherein a piston is fixed to a movable member having a cam follower positioned on said member, and that the drive means is connected to a rotatable cam having a highest point and a lowermost point immediately following said highest point, which cam cooperates with said cam follower, so as to cause that rotation of the cam is converted into longitudinal movement of the member and the piston that is fixed to said member.

7. A needle-less injector according to any one of claim 1 to 6 wherein the dispensing members are operated by a single trigger (5).

8. A needle-less injector according to any one of claim 1 to 7 wherein the needleless injector comprises a single drive means for actuating said injector.

9. A needle-less injector according to any one of claim 1 to 8 wherein each of said chambers has a liquid inlet which is arranged to allow liquid to enter into the chamber when desired.

10. A needle-less injector according to claim 9 wherein a supply-line is provided for letting in liquid into each of the chambers and wherein a sensor for detecting the presence of liquid for injecting purposes is present that is able to detect liquid in the supply-line, and that the operation of the drive means is dependent on the sensor.

11. A needle-less injector according to claim 9 or 10 wherein the drive means is enabled to block the liquid outlet when the sensor fails to detect liquid for injecting purposes.

12. A needle-less injector according to any one of claim 1 to 11 wherein the injector comprises a pressure sensor (12) in order to sense axial pressure, which pressure sensor is connected to a first switching means within the housing which is connected to the drive means and a power source for said drive means, and which first switching means is capable to establish contact between the drive means for actuating said injector and the power source when the pressure sensor is actuated by a selected amount of axial pressure.

13. A needle-less injector according to any one of claim 1 to 12 including a trigger switch, which trigger switch is connected to a second switching means within the housing which is connected to the drive means and a power source for said drive means **characterized in that** in the actuated state of the trigger switch the second switching means is enabled to establish contact between the power source and the drive means for actuating the injector and which second switching means is capable to establish contact between the drive means for actuating said injector and the power source when the trigger switch is actuated by a selected amount of pressure on the trigger switch.

## Patentansprüche

1. Nadelloser Injektor mit einem Gehäuse (2, 3), umfassend:
zwei oder mehr separate Kammern (11), die in dem Injektor definiert sind, um zu injizierende Flüssigkeit zu enthalten;
- wobei jede Kammer umfasst:
- einen Flüssigkeitsauslass (1), der am vorderen Ende des Injektors positioniert ist; und
- ein Abgabeelement in Kontakt mit der Flüssigkeit in den Kammern, und beweglich in einer ersten Richtung, um das Volumen der Kammer zu reduzieren, um zu bewirken, dass die darin enthaltene Flüssigkeit durch den Flüssigkeitsauslass ausgestoßen wird;
wobei der nadellose Injektor des Weiteren ein Antriebsmittel zum Betätigen des Injektors umfasst,
**dadurch gekennzeichnet, dass** der Abstand zwischen dem Flüssigkeitsauslass für jede der Kammern mindestens 20 mm beträgt.

2. Nadelloser Injektor nach Anspruch 1, wobei der Abstand zwischen dem Flüssigkeitsauslass für jede der Kammern mindestens 29 mm beträgt.

3. Nadelloser Injektor nach einem der Ansprüche 1 oder 2, wobei der Abstand zwischen dem Flüssigkeitsauslass für jede der Kammern mindestens 32 mm beträgt.

4. Nadelloser Injektor nach einem der Ansprüche 1 bis 3, wobei der Abstand zwischen dem Flüssigkeitsauslass für jede der Kammern höchstens 100 mm ist.

5. Nadelloser Injektor nach einem der Ansprüche 1 bis 4, wobei das Abgabeelement ein federbelasteter Kolben ist, der in der Kammer durch die Feder in eine erste Richtung zu einer bevorzugten Position bewegbar ist, in der das Volumen der Kammer auf einem Minimum ist und die Feder nahezu unbelastet ist, und wobei der Kolben in eine zweite Richtung entgegengesetzt zu der ersten Richtung beweglich ist, indem das Antriebsmittel betätigt wird, während einer Kraft seitens der Feder entgegengewirkt wird und der Kolben in eine nichtbevorzugte Position bewegt wird, in welcher die Feder belastet ist.

6. Nadelloser Injektor nach einem der Ansprüche 1 bis 5, wobei ein Kolben an einem bewegbaren Element mit einem Nockenstößel, der auf dem Element positioniert ist, fixiert ist, und wobei das Antriebsmittel mit einem rotierbaren Nocken verbunden ist, der einen höchsten Punkt und einen niedrigsten Punkt, welcher dem höchsten Punkt unmittelbar folgt, aufweist, wobei der Nocken mit dem Nockenstößel zusammenarbeitet, um so zu bewirken, dass eine Rotation des Nockens in eine Längsbewegung des Elements und des Kolbens, der an dem Element fixiert ist, konvertiert wird.

7. Nadelloser Injektor nach einem der Ansprüche 1 bis 6, wobei die Abgabeelemente durch einen einzelnen Auslöser (5) betrieben werden.

8. Nadelloser Injektor nach einem der Ansprüche 1 bis 7, wobei der nadellose Injektor ein einzelnes Antriebsmittel zum Betätigen des Injektors umfasst.

9. Nadelloser Injektor nach einem der Ansprüche 1 bis 8, wobei jede der Kammern einen Flüssigkeitseinlass aufweist, der angeordnet ist, um das Eintreten von Flüssigkeit in die Kammer zuzulassen, wenn dies gewünscht ist.

10. Nadelloser Injektor nach Anspruch 9, wobei eine Zuführleitung bereitgestellt wird, um Flüssigkeit in jede der Kammern einzulassen, und wobei ein Sensor zum Detektieren der Anwesenheit von Flüssigkeit zu Injektionszwecken vorhanden ist, der in der Lage ist, Flüssigkeit in der Zuführleitung zu detektieren, und wobei der Betrieb des Antriebsmittels von dem Sensor abhängt.

11. Nadelloser Injektor nach Anspruch 9 oder 10, wobei das Antriebsmittel befähigt ist, den Flüssigkeitsauslass zu blockieren, wenn der Sensor keine Flüssigkeit für Injektionszwecke detektieren kann.

12. Nadelloser Injektor nach einem der Ansprüche 1 bis 11, wobei der Injektor einen Drucksensor (12) umfasst, um axialen Druck abzufühlen, wobei der Drucksensor mit einem ersten Schaltmittel innerhalb des Gehäuses verbunden ist, das mit dem Antriebsmittel und einer Leistungsquelle für das Antriebsmittel verbunden ist, und wobei das erste Schaltmittel in der Lage ist, Kontakt zwischen dem Antriebsmittel zur Betätigung des Injektors und der Leistungsquelle herzustellen, wenn der Drucksensor durch eine ausgewählte Menge an axialem Druck betätigt wird.

13. Nadelloser Injektor nach einem der Ansprüche 1 bis 12, der einen Auslöserschalter einschließt, wobei der Auslöserschalter mit einem zweiten Schaltmittel innerhalb des Gehäuses verbunden ist, das mit dem Antriebsmittel und einer Leistungsquelle für das Antriebsmittel verbunden ist, **dadurch gekennzeichnet, dass** in dem betätigten Zustand des Auslöserschalters das zweite Schaltmittel befähigt wird, Kontakt zwischen der Leistungsquelle und dem Antriebsmittel herzustellen, um den Injektor zu betätigen, und wobei das zweite Schaltmittel in der Lage ist, Kontakt zwischen dem Antriebsmittel zur Betätigung des Injektors und der Leistungsquelle herzustellen, wenn der Auslöserschalter durch eine ausgewählte Menge an Druck auf den Auslöserschalter betätigt wird.

## Revendications

1. Injecteur sans aiguille ayant un boîtier (2, 3) comprenant :
deux ou plus de deux chambres séparées (11) définies à l'intérieur dudit injecteur pour contenir le liquide à injecter ;
- chaque chambre comprenant :
- une sortie de liquide (1) positionnée à l'extrémité avant de l'injecteur ; et
- un élément de distribution en contact avec le liquide dans lesdites chambres et mobile dans une première direction pour réduire le volume de ladite chambre afin de provoquer l'expulsion du liquide contenu dans celle-ci à travers ladite sortie de liquide ;
l'injecteur sans aiguille comprenant en outre un moyen d'entraînement pour actionner ledit injecteur,
**caractérisé en ce que** la distance entre la sortie de liquide pour chacune desdites chambres est d'au moins 20 mm.

2. Injecteur sans aiguille selon la revendication 1, la distance entre la sortie de liquide pour chacune desdites chambres étant d'au moins 29 mm.

3. Injecteur sans aiguille selon l'une quelconque des revendications 1 ou 2, la distance entre la sortie du liquide pour chacune desdites chambres étant d'au moins 32 mm.

4. Injecteur sans aiguille selon l'une quelconque des revendications 1 à 3, la distance entre la sortie du liquide pour chacune desdites chambres étant d'au plus 100 mm.

5. Injecteur sans aiguille selon l'une quelconque des revendications 1 à 4, l'élément de distribution étant un piston chargé par ressort, déplaçable dans ladite chambre par le ressort dans la première direction jusqu'à une position préférée à laquelle le volume de ladite chambre est minimal et le ressort est presque déchargé, et lequel piston étant déplaçable dans une seconde direction opposée à la première direction par actionnement du moyen d'entraînement tout en contrecarrant une force du ressort et en déplaçant le piston jusqu'à une position non préférée à laquelle le ressort est chargé.

6. Injecteur sans aiguille selon l'une quelconque des revendications 1 à 5, un piston étant fixé à un élément mobile ayant un suiveur de came positionné sur ledit élément, et le moyen d'entraînement étant relié à une came rotative ayant un point le plus haut et un point le plus bas suivant immédiatement ledit point le plus haut, laquelle came coopère avec ledit suiveur de came, de manière à faire en sorte que la rotation de la came soit convertie en un mouvement longitudinal de l'élément et du piston qui est fixé audit élément.

7. Injecteur sans aiguille selon l'une quelconque des revendications 1 à 6, les éléments de distribution étant actionnés par une seule gâchette (5).

8. Injecteur sans aiguille selon l'une quelconque des revendications 1 à 7, l'injecteur sans aiguille comprenant un seul moyen d'entraînement pour actionner ledit injecteur.

9. Injecteur sans aiguille selon l'une quelconque des revendications 1 à 8, chacune desdites chambres ayant une entrée de liquide qui est agencée pour permettre au liquide d'entrer dans la chambre lorsque cela est souhaité.

10. Injecteur sans aiguille selon la revendication 9, une conduite d'alimentation étant prévue pour laisser entrer du liquide dans chacune des chambres, et un capteur pour détecter la présence de liquide à des fins d'injection étant présent qui est capable de détecter du liquide dans la conduite d'alimentation, et dont le fonctionnement des moyens d'entraînement dépend du capteur.

11. Injecteur sans aiguille selon la revendication 9 ou 10, le moyen d'entraînement étant activé pour bloquer la sortie de liquide lorsque le capteur n'arrive pas à détecter de liquide à des fins d'injection.

12. Injecteur sans aiguille selon l'une quelconque des revendications 1 à 11, l'injecteur comprenant un capteur de pression (12) afin de détecter une pression axiale, lequel capteur de pression étant connecté à un premier moyen de commutation à l'intérieur du boîtier qui est connecté au moyen d'entraînement et à une source d'alimentation pour ledit moyen d'entraînement, et lequel premier moyen de commutation étant capable d'établir un contact entre le moyen d'entraînement pour actionner ledit injecteur et la source d'alimentation lorsque le capteur de pression est actionné par une quantité sélectionnée de pression axiale.

13. Injecteur sans aiguille selon l'une quelconque des revendications 1 à 12, comprenant un commutateur à gâchette, lequel commutateur à gâchette est relié à un second moyen de commutation à l'intérieur du boîtier qui est relié au moyen d'entraînement et à une source d'alimentation pour ledit moyen d'entraînement, **caractérisé en ce que** dans l'état actionné du commutateur à gâchette, le second moyen de commutation est autorisé à établir un contact entre la source d'alimentation et le moyen d'entraînement pour actionner l'injecteur, et lequel second moyen de commutation est capable d'établir un contact entre le moyen d'entraînement pour actionner ledit injecteur et la source d'alimentation lorsque le commutateur à gâchette est actionné par une quantité sélectionnée de pression sur le commutateur à gâchette.
